(19) Europäisches Patentamt
European Patent Office
Office européen des brevets

(11) Publication number: 0 154 149
B1

(12) **EUROPEAN PATENT SPECIFICATION**

(45) Date of publication of patent specification: **23.08.89**

(51) Int. Cl.$^4$: **C 07 D 499/00,** A 61 K 31/43 // C07D205/08, C07F7/18

(21) Application number: **85100821.9**

(22) Date of filing: **26.01.85**

(54) **Hydrazone substituted penems.**

(30) Priority: **02.02.84 US 576499**
**01.03.84 US 585314**

(43) Date of publication of application:
**11.09.85 Bulletin 85/37**

(45) Publication of the grant of the patent:
**23.08.89 Bulletin 89/34**

(84) Designated Contracting States:
**AT BE CH DE FR GB IT LI NL SE**

(56) References cited:
**EP-A-0 002 210**
**EP-A-0 035 188**
**EP-A-0 087 792**
**EP-A-0 146 730**

**The file contains technical information submitted after the application was filed and not included in this specification**

(73) Proprietor: **SCHERING CORPORATION**
**2000 Galloping Hill Road**
**Kenilworth New Jersey 07033 (US)**

(72) Inventor: **Girijavallabhan, Viyyoor M.**
**10 Maplewood Drive**
**Parsippany, N.J. 07054 (US)**
Inventor: **Ganguly, Ashit K.**
**96 Cooper Avenue**
**Upper Montclair, N.J. 07043 (US)**
Inventor: **Pinto, Patrick A.**
**232 Randolph Avenue**
**Mine Hill, N.J. 07801 (US)**
Inventor: **Versace, Richard W.**
**230 Lakeview Drive**
**Ringwood, N.J. 07456 (US)**
Inventor: **Patel, Naginbhai M.**
**121 Tappan Street**
**Kearny, N.J. 07032 (US)**

(74) Representative: **Ritter, Stephen David et al**
**Mathys & Squire 10 Fleet Street**
**London EC4Y 1AY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 154 149 B1

## Description

This invention relates to 2-(hydrazono-alkylthio)-penems and their pharmaceutically acceptable salts and esters, which compounds possess potent antibacterial activity.

Derivatives of 2-alkylthiopenems having imino-substituents on the 2-alkylthio group are disclosed in EP—A—0087792, EP—A—0035188, EP—A—0002210 and EP—A—0146730 (which falls under Article 54(3) EPC).

There is a continuing need for new antibacterial agents because continued extensive use of effective antibacterials gives rise to resistant strains of pathogens.

The novel penem compounds of this invention are represented by the formula:—

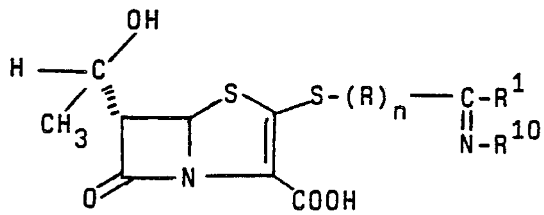

I

and pharmaceutically acceptable salts and pharmaceutically acceptable esters thereof, in racemic or optically active forms wherein

n is one or two;

R represents

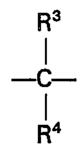

wherein $R^3$ and $R^4$ are independently selected from hydrogen, pyrrole, lower alkyl, hydroxy-lower alkyl, halo-lower alkyl, amino-lower alkyl, carbamido-lower alkyl, cyano-lower alkyl, carbamoyloxylower alkyl, carbamoyl-lower alkyl, carboxy-lower alkyl, heterocyclyl-lower alkyl wherein the heterocyclic moiety has 5 or 6 ring atoms, at least one of which is carbon, at least one is nitrogen, sulfur or oxygen, and the remaining ring atoms are independently selected from carbon, nitrogen, oxygen and sulfur;

$R^1$ represents hydrogen, loweralkoxycarbonylcarbonyl, carboxy, lower alkyl, hydroxy-lower alkyl, amino-lower alkyl, carboxy-lower alkyl, carbamoyloxy-lower alkyl, carbamoyl-lower alkyl, haloloweralkyl, cyano-lower alkyl, heterocyclyl or heterocyclyl-lower alkyl wherein the heterocyclic moieties have 5 or 6 ring atoms, at least one of which is carbon, at least one is nitrogen, sulfur or oxygen, and the remaining ring atoms are independently selected from oxygen, carbon, nitrogen and sulfur;

$R^{10}$ represents

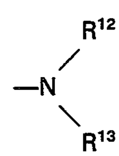 ;

$R^{12}$ and $R^{13}$ independently represent hydrogen, carbamoyl, lower alkyl, carboxy-loweralkyl, hydroxy-lower alkyl, amino-lower alkyl, imidazolyl-lower alkyl, triazolyl-lower alkyl, tetrazolyl-lower alkyl, pyridinium-lower alkyl, aryl, aryl-lower alkyl or $R^{12}$ and $R^{13}$ together with the N to which they are attached form a heterocyclic ring contaioning 5 or 6 atoms, at least one of which is carbon and the remaining ring atoms are independently selected from nitrogen, carbon, oxygen and sulfur wherein said heterocyclic moiety is unsubstituted or substituted with lower alkyl, hydroxy-lower alkyl, amino-lower alkyl, carboxy-lower alkyl, carboxyl, amino, hydroxy, imidazole-lower alkyl, triazole-lower alkyl, tetrazole-lower alkyl or pyridinium-lower alkyl, or when said heterocyclic moiety is 1,4-thiazane, said 1,4-thiazane moiety can also be substituted by two oxo-groups attached to the sulfur atom.

As used herein, the term "lower alkyl" means straight or branched chain alkyl groups of 1 to 6 carbon atoms, e.g. methyl, ethyl, propyl, iso-propyl, butyl, t-butyl, pentyl, neopentyl or hexyl; "halo" means fluorine, chlorine, bromine or iodine, with fluorine being preferred; "heterocyclyl" unless otherwise stated means a saturated, unsaturated or aromatic heterocyclic group with 5 or 6 ring atoms, at least one of which is carbon, at least one is oxygen, nitrogen or sulfur and the remaining ring atoms are independently selected from carbon, nitrogen, oxygen and sulfur. The preferred heteorcyclics contain a nitrogen atom and are bonded to the molecule through a nitrogen atom.

Preferred heterocyclyl groups within the scope of this invention are, for example, pyrrole, imidazole, piperidine, pyrrolidine, imidazolidine, pyrazole, triazole, isothiazole, pyridinium, tetrazole, thiadiazole or thiazole. All position isomers of the heterocyclics are contemplated for examples, 1,2,4-triazole, 4,1,2-triazole, 1,2,3-triazole, 2,1,3-triazole, 1,3-thiazole, 1,2,3,4-tetrazole or 2,1,3,4-tetrazole.

The most preferred heterocyclics are imidazole, 1,2,4-triazole, 1,2,3-triazole, 1,3-thiazole and imidazolidine.

"Aryl" as used herein means a phenyl either substituted or unsubstituted wherein said substituents are selected from hydrogen, lower alkyl, hydroxy-lower alkyl, carboxy-lower alkyl, carboxyl or amino.

The preferred definition for $R^1$ is hydrogen or lower alkyl, and for R the group

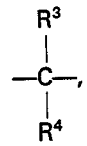

the preferred meaning of $R^3$ and $R^4$ is hydrogen.

One preferred group of compounds are those wherein $R^{12}$ and $R^{13}$ independently represent hydrogen or carbamoyl, or together with the nitrogen atom to which they are attached $R^{12}$ and $R^{13}$ form a heterocyclic ring as defined above. More preferred in this group are compounds wherein n is 1, $R^{12}$ and $R^{13}$ form a triazole ring or $R^{12}$ is hydrogen and $R^{13}$ is carbamoyl.

"Pharmaceutically acceptable salts" as used herein means alkali metal salts such as sodium and potassium salts; alkaline earth metal salts such as calcium, magnesium and aluminium salts; amine salts formed from a wide variety of suitable organic amines, e.g., aliphatic, cycloaliphatic, (cycloaliphatic)aliphatic or araliphatic primary, secondary or tertiary mono-, di- or polyamines, or heterocyclic bases, e.g., salts derived from triethylamine, 2-hydroxyethylamine, di-(2-hydroxyethyl)amine, tri-(2-hydroxyethyl)amine, 4-aminobenzoic acid-2-diethylaminoethyl ester, 1-ethylpiperidine, bicyclohexylamine, N,N'-dibenzylethylenediamine, pyridine, collidine, quinoline, procaine, dibenzylamine, 1-ephenamine and N-alkylpiperidine. Acid addition salts are formed from mineral acids such as hydrochloric, hydrobromic, hydroiodic, phosphoric or sulfuric acids, or are formed from organic carboxylic or sulfonic acids such as trifluoroacetic, para-toluene sulfonic, maleic, acetic, citric, oxalic, succinic, benzoic, tartaric, fumaric, mandelic, ascorbic and malic acids. The compounds of this invention which contain a 3-carboxylic group and a basic group (the heterocyclic group) form an inner salt, i.e., a Zwitterion.

"Pharmaceutically acceptable esters" means physiologically cleavable esters, i.e., metabolizable esters known in the penicillin, cephalosporin and penem arts to be easily cleaved within the body to the parent acid. Examples of such esters are indanyl, phthalidyl, methoxymethyl, glycyloxymethyl, phenylglycyloxymethyl, thienylglycyloxymethyl, acetoxymethyl and pivaloyloxymethyl.

Preparation of the foregoing salts and esters may be carried out according to conventional procedures for forming salts of beta-lactams such as penicillins, cephalosporins and penems. Salts can be formed, for example, by treating with metal compounds such as alkali metal salts of suitable carboxylic acids, or with ammonia or a suitable organic amine, wherein preferably stoichiometric amounts or only a small excess of the salt-forming agent is used. Acid addition salts are obtained in the usual manner, for example, by treating with an acid or a suitable anion exchange reagent. Inner salts of the compounds of formula I, i.e., a zwitterion, may be formed by neutralizing salts such as acid addition salts to the isoelectric point. The esters are preparable in a manner analogous to the preparation of the corresponding esters of penicillins and cephalosporins.

Salts may be converted in the usual manner into the free carboxy compounds.

Compounds of this invention possess 3 or more asymmetric carbon atoms indicated in the partial formula I(a) below at the 5, 6 and 8 and the 2' and 3'-position carbon atoms

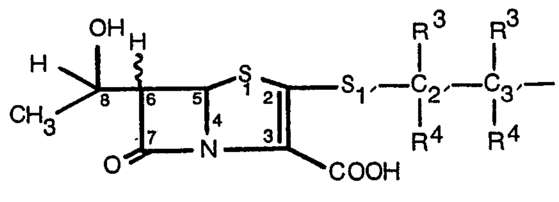

I(a)

At the 5,6 and 8 positions, compounds of the invention may possess 5R, 6S, 8R or 5R, 6R, 8S stereochemistry at those chiral atoms. The preferred absolute stereochemistry for the compounds of the present invention at those positions is 5R, 6S, 8R.

Compounds of this invention wherein $R^3$ and $R^4$ on the carbon atoms are different will have additional asymmetric carbon atom(s) as shown in formula I(a) at the 2' and 3' positions. All the possible resulting stereo-isomers are included herein.

When tested in standardized microbiological assays, the compounds of this invention are active against such gram-positive organisms as *Staphylococcus epidermis* and *Bacillus subtilis*, and such gram-

negative organisms as *E. coli* and *Salmonella* at test levels of 0.03 to 2.0 micrograms/ml. Additionally, they show activity against organisms which produce beta-lactamases, e.g., penicillinase and cephalosporinase, indicating a resistance against these enzymes.

The compounds of this invention exhibit low protein binding and their metabolites have little or no unpleasant odor.

As antibacterial agents, the compounds of this invention are conventionally formulated for oral, parenteral, topical and transdermal use. Thus, this invention includes within its scope pharmaceutical compositions comprising the compounds of this invention in admixture with a pharmaceutically acceptable carrier therefor. In addition, the present invention also provides a method of preparing a pharmaceutical composition for treating bacterial infections in animals, particularly warm-blooded animals including humans, having a susceptible bacterial infection by administering to said animal an antibacterially effective amount of a compound of this invention, or a pharmaceutical composition thereof. In the foregoing compositions, the compounds of this invention can be used as the sole active antibacterial agent or in combination with other antibacterial agents and/or enzyme inhibitors.

For oral administration, the compounds of this invention are typically formulated in the form of tablets, capsules, elixirs, or the like. For parenteral administration, they may be formulated into solutions or suspensions. Typical topical formulations are those such as lotions, creams, ointments, sprays, and formulations intended for use in mechanical delivery devices, e.g., transdermal. Parenteral administration is preferred.

Typical pharmaceutically acceptable carriers for use in the formulations described above are exemplified by: sugars such as lactose, sucrose, mannitol and sorbitol; starches such as corn starch, tapioca starch and potato starch; cellulose and derivatives such as sodium carboxymethyl-cellulose, ethylcellulose and methyl-cellulose; calcium phosphates such as dicalcium phosphate and tri-calcium phosphate; sodium sulfate; calcium sulfate; polyvinyl-pyrrolidone; polyvinyl alcohol; stearic acid; alkaline earth metal stearates such as magnesium stearate; vegetable oils such as peanut oil, cottonseed oil, sesame oil, olive oil and corn oil; non-ionic, cationic and anionic surfactants; ethylene glycol polymers; betacyclodextrin; fatty alcohols, hydrolyzed cereal solids; water; polyalkylene glycols; gums; and petrolatum; as well as other non-toxic compatible fillers, binders, disintegrants and lubricants commonly used in pharmaceutical formulations. The compositions may also contain preservatives, aerosol propellants and coloring, thickening, suspending, dispensing, emulsifying, wetting, stabilizing and buffering agents.

The dosage of the compounds of this invention which is administered is dependent, in the judgement of the attending clinician, upon a variety of factors, i.e., the age and weight of the individual being treated, the mode of administration, the potency of the administered compound and the type and severity of the bacterial infection being prevented or reduced. Typically, the dosage administered per day will be in the range of from about 1 to 250 mg/kg and preferably from about 5 to 20 mg/kg in divided dosages. Typically, the dosage will be administered in dosage units containing convenient amounts, for example, 125, 250 or 500 mg of active ingredient combined with a suitable physiologically acceptable carrier or diluent.

The compounds of this invention may be prepared by reacting a tautomeric compound of the formula

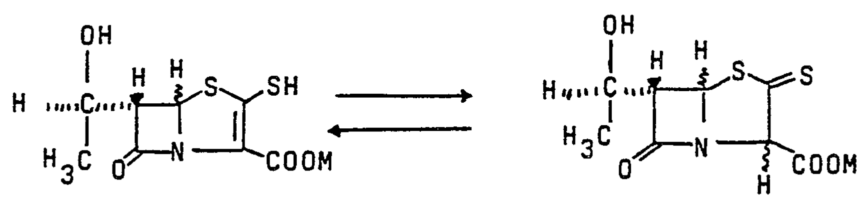

IX(a) IX(b)

wherein M represents a protecting group with a compound of the formula

$$L-(R)_n-\underset{\underset{\displaystyle N-R^{10}}{\|}}{C}-R^1 \qquad XII$$

wherein n, R, $R^1$ and $R^{10}$ are as previously defined and L represents a leaving group, followed by deprotection of the carboxyl group, and if desired, by esterification of said carboxy group or by transformation into a salt.

The tautomeric compound of formula IXa and IXb is a known compound and its preparation is described in published European Patent Application No. 82110612.7 (Publication No. 0080162, 01.06.83) as well as in published Japanese Patent Application No. 74762/1980. A preferred method for making the tautomeric compound is illustrated in Example I.

Preferred carboxy protecting groups M are $-CH_2CH_2R^6$ or $-CH_2CH=CH_2$, with $R^6$ being a silyl group such as trimethylsilyl or t-butyldiphenylsilyl, a cyano or arylsulfonyl group. Preferably M is $-CH_2CH=CH_2$.

The preferred leaving groups represented by L are halogen (bromine or chlorine) and trifluoromethanesulfonyl.

The reaction of the tautomeric compound with a compound of formula XII is preferably carried over in an inert organic solvent such as tetrahydrofuran under an inert atmosphere, e.g. nitrogen. The reaction is completed in about 1 to 3 hours.

The removal of the protecting group M is preferably carried out by treatment with an alkali base in the presence of a catalyst. If the product of formula I is a Zwitterion, the deprotection requires only the catalyst or any mild nucleophil, e.g. water or alcohol.

When M represents an allyl group the removal thereof is preferably effected by the addition of the protected compound to a solution containing palladium (zero) and an alkali alkylcarboxylate, carboxylic acid or aqueous carbonate. This method is described by McCombie in U.S. Patent 4,314,942. Under these conditions, the removal of the allyl group and formation of the alkali salt or the free acid of the compound occurs.

The compounds of formula XII in which $R^{10}$ is —$OR^2$ are either known compounds or are prepared from known compounds by conventional processes for preparing oximes by reacting the appropriate aldehyde or ketone with a hydroxyl amine or ammonium hydroxide. Generally the process is carried out as follows:

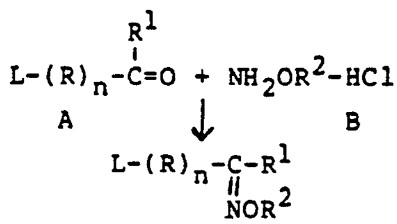

XII

In the above formulae L, N, R, $R^1$ and $R^2$ are as defined hereinabove.

The compounds of formula XII in which $R^{10}$ is

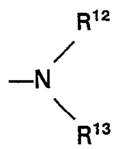

are either known compounds or are prepared from known compounds by conventional processes for preparing hydrazones by reacting the appropriate aldehyde or ketone with a hydrazino compound or an N-amino-substituted nitrogen-containing heterocyclic compound. Generally the process is carried out as follows:

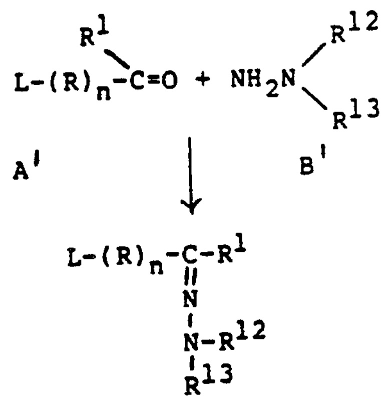

XII

wherein L, n, R, $R^1$, $R^{12}$ and $R^{13}$ are as defined hereinabove.

The following examples illustrate the preparation of compounds and compositions of this invention.

### Example 1

Preparation of (5R,6S,8R)allyl-2-thiol-6-(1-hydroxyethyl)penem-3-carboxylate and (5R,6S,8R)allyl-2-thiocarbonyl-6-(1-hydroxyethyl)penam-3-carboxylate

(A) Preparation of (3S,4R)-1-(allyloxycarbonylmethyl)-3-(1-hydroxyethyl)-4-(triphenylmethylthio)azetidin-2-one

Add 3 gm of (3S,4R)-3-(1-hydroxyethyl)-4-(triphenylmethylthio)azetidin-2-one to 10 ml of acetonitrile containing 0.286 g of cesium carbonate. Add 0.2 g of alpha-iodo-allyl acetate to the system. Stir the system

at room temperature for 16 hours. Dilute with ether (50 ml), filter and wash the ether layer with 1% aqueous phosphoric acid, followed by water. After drying over sodium sulfate remove solvent to give a foamy solid.

(B) Preparation of (3S,4R)-3-(1-hydroxyethyl)-1-allyloxycarbonylmethyl-4-sulfhydryl-azetidin-2-one

Add 500 mg of (3S,4R)-1-(allyloxycarbonylmethyl-3-(1-hydroxyethyl)-4-triphenylmethylthio)azetidin-2-one and 20 ml tetrahydrofuran to a 50 ml flask. Add zinc dust and 10% hydrochloric acid in small portions over 1 hour until all of the starting material is reacted. Recover the product by filtering off the excess zinc and removing the solvent to crystallize the title product.

NMR: ($CDCl_3$) =6.2—5.7 (1H, m); 5.5—5.15 (2H, m); 5.0 (1H, dd, J=3.9 c/s); 4.75—4.55 (2H,m); 4.45—3.95 (1H,m); 4.14(1H, d, J=18 c/s); 3.78 (1H, d, J=18 c/s); 3.19(1H, dd, J=6,3 c/s); 2.09 (1H, d, J=9 c/s); 1.34(3H, d, J=6 c/s).

(C) Preparation of (3S,4R)-3-(1-trimethylsilyloxyethyl)-1-allyloxycarbonylmethyl-4-sulfhydryl-azetidin-2-one

Add the entire amount of (3S,4R)-3-(1-hydroxyethyl)-1-allyloxycarbonylmethyl-4-sulfhydryl-azetidin-2-one produced in step (B) above to 25 ml of methylene chloride. To this system add 1.1 ml of bis-silylacetamide. Stir the system at room temperature for 15 minutes to give the title compound.

(D) Preparation of (3S,4R)-1-(allyloxycarbonylmethyl)-3-(1-trimethylsilyloxyethyl)-4-(1′-imidazolylthiocarbonylthio)azetidin-2-one

After completion of step (C) above and to the same solution add 350 mg of thiocarbonyldiimidazole. Stir the system at room temperature for 3 hours. Filter the solution and wash the precipitate with methylene chloride. Collect the filtrate and remove the methylene chloride by stripping. Chromatograph the residue on silica gel eluting with 20% ethyl acetate/methylene chloride to yield 335 mg of the title compound.

NMR: 8.4, 1H, s; 7.65, 1H, d(J=1Hz); 7.05, 1H (dJ=Hz); 5.95, 1H, d (J=2Hz); 5.8, 1H, m; 5.45—5.1, 2H, m; 4.3, 1H, m; 4.1, 2H, Q(J=16Hz); 3.5, d,d (J=2,6); 1.35; 3H, d (J=6Hz).

(E) Preparation of (5R,6S,8R) allyl-2-thiol-6-(1-trimethylsilyloxyethyl)penem-3-carboxylate and (5R,6S,8R) allyl-2-thiocarbonyl-6-(1-trimethylsilyloxyethyl)penam-3-carboxylate

Add 170 mg of (3S,4R)-1-(allyloxycarbonylmethyl)-3-(1-trimethylsilyloxyethyl)-4-(1′-imidazolylthiocarbonylthio)azetidin-2-one to 40 ml of anhydrous tetrahydrofuran under a nitrogen atmosphere. Cool the system to −78°C and then add 0.6 ml of 1 M lithium di(trimethylsilyl)-amine in hexane dropwise to the system. Stir the system at −78°C for 5 minutes. Add 0.2 ml of acetic acid to the system. Dilute the system to 200 ml with methylene chloride. Wash the organic solution with water, aqueous sodium bicarbonate solution and again with water. Purify the product by chromatography by rapidly eluting the sample through silica gel with 5% ethyl acetate/methylene chloride to afford 125 mg of the desired products and the desilylated products.

(F) Preparation of (5R,6S,8R) allyl-2-thiol-6-(1-hydroxyethyl)penem-3-carboxylate and (5R,6S,8R) allyl-2-thiocarbonyl-6-(1-hydroxyethyl)penam-3-carboxylate

To a 25 ml flask add the entire mixture produced in step (E) along with 5 ml of tetrahydrofuran, 1 ml of water and 1 ml of acetic acid. Stir the system at room temperature for 2 hours. Add ethyl acetate to the solution and wash the organic phase with sodium bicarbonate solution, water and then brine. Dry the organic phase over anhydrous sodium sulfate, filter and remove the solvent by stripping to give the title compound.

## Example 2

(5R,6S,8R)sodium-2-[2-(1,2,4-triazol-4-ylimino)propylthio]-6-(1-hydroxyethyl)penem-3-carboxylate

(A). Add 3.6 ml Cl—$CH_2COCH_3$ to a mixture of 3.0 g 4-aminotriazole, 150 ml distilled THF and 3.8 ml acetic acid and stir at room temperature for about 7 hours. Add more Cl—$CH_2COCH_3$ (3 ml) and continue stirring overnight, about 15 hours. Concentrate the reaction mixture to a thick colorless oily residue and dissolve in 100 ml $CH_3CN$. Filter through a small silica gel column and elute with $CH_3CN$ to obtain 2-(1,2,4-triazol-4-ylimino)-1-chloropropane as shown by NMR.

(B). Add a solution of 1.2 g chlorohydrazone in 5 ml tetrahydrofuran (THF) and 5 ml water to a mixture of 2 g of the compounds prepared in Example 1(F) 20 ml THF and 5 ml water. Adjust the pH of the reaction mixture to about 8.0 with $NaHCO_3$. The reaction is completed in about one-half hour as evidenced by TLC (20% $CH_3CN/CH_2Cl_2$). Remove the THF solvent then add 100 ml $CH_2Cl_2$ and dry over sodium sulfate. Purify by filtering through a silica column and eluting with $CH_3CN$ to obtain the allyl ester of the title compound.

(C). Add 0.12 ml 2-ethyl-hexanoic acid and 50 mg triphenyl-phosphine to a solution of 100 mg of the allyl ester prepared in (B) above in 5 ml dry $CH_2Cl_2$. Add 20 mg Pd-[$P(C_6H_5)_3$]$_4$ to the resulting solution. The reaction is completed in about one-half hour as confirmed by TLC (20% $CH_3CN$/ethyl acetate). Add 40 mg sodium hexanoate in ethylacetate to obtain a precipitate of the title compound as a hydroscopic solid.

NMR: ($D_2O$) δ8.5(s,2H), 5.57 (s,1H), 3.7—4.25 (m,4H), 2.1(s,3H), 1.2(d,3H).
Mass spectrum: FAB$[M+H]^+=370$,, $[M+Na]^+=392$.

Following the procedures of Example 2 but replacing 4-aminotriazole with imidazol-2-yl-hydrazine gives sodium(5R,6S,8R)-2-[2-(imidazol-2-yl hydrazono)propylthio]-6-(1-hydroxyethyl)penem-3-carboxylate.

## Example 3

(5R,6S,8R)sodium-2-[2-($N^2$-carbamoylhydrazino)propylthio]-6-(1-hydroxyethyl)penem-3-carboxylate

(A). Add 1.0 g chloroacetone and a solution of 0.84 g $NaHCO_3$ in 10 ml water to a solution of 1.1 g semicarbazide hydrochloride in 25 ml THF and stir overnight. Two layers form. Separate the top layer and concentrate to obtain an off-white solid, 2-semicarbazono-1-chloropropane.

(B). Add 1.2 g of the semicarbazone prepared in A above in 5 ml water to a mixture of 2.0 g of the compounds prepared in Example 1(F) 30 ml THF, 10 ml water and 0.6 g $NaHCO_3$. Stir the mixture for about one-half hour to complete the reaction as evidenced by TLC (20% $CH_3CN$/ethylacetate). Remove the THF solvent to obtain a solid. Work up the solid and recover the allyl ester of the title compound.

(C). Add 10 mg $Pd[P(C_6H_5)_3]_4$ to a solution of 100 mg of the allyl ester prepared in B above, 50 mg triphenyl phosphine and 0.2 ml 2-ethylhexanoic acid in 10 ml THF. After one-half hour add 40 mg Na-ethylhexanoate to cause a precipitate to gradually form. When the precipitation stops the reaction is completed. Remove the THF solvent at room temperature and add 10 ml ethylacetate. Filter off the solid and work up, then lyophilize to obtain the title compound as a white solid.

NMR($D_2O$): δ5.3(d,1H), 3.9(m,1H), 3.55(d of d,1H), 1.7(s,3H), 1.0(d,3H)
Mass spectrum: FAB. $[M+H]^+=361$, $[M+Na]^+=383$.

Following the procedures of Examples 2 and 3, but replacing the starting compounds with appropriately substituted analogous reagents A' and B' which are known or can be prepared by methods known in the art for preparing analogous compounds, the compounds of formula I are prepared. The following table shows the hydrazone moiety which results when reagent A' is

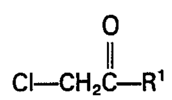

and reagent B' is as in the table.

TABLE

| Reagent B' | Hydrazone |
|---|---|
| 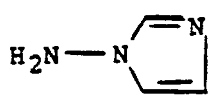 | 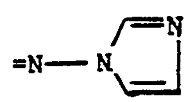 |
| $H_2N—NH-CH_2CH_2OH$ | $=N—NHCH_2CH_2OH$ |
| 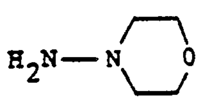 | 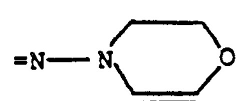 |
| 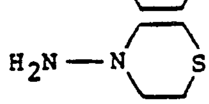 | 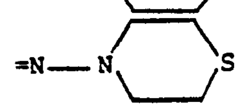 |
| 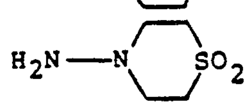 | 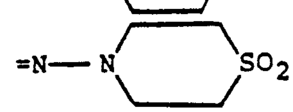 |
| 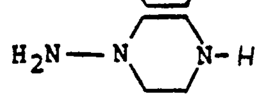 | 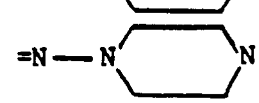 |

TABLE (continued)

| Reagent B' | Hydrazone |
|---|---|
| 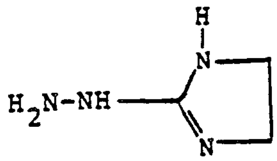 | 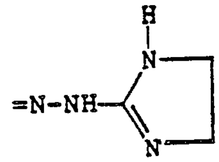 |
| 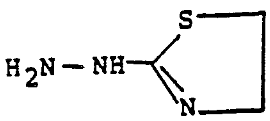 | 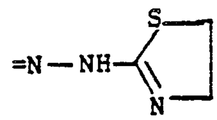 |
| 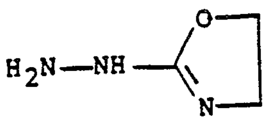 | 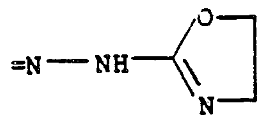 |
| 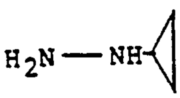 | 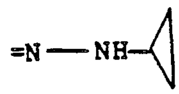 |
| $H_2N-NH-CH_3$ | $=N-NH-CH_3$ |
| $H_2N-NH-CH_2CH_3$ | $=N-NH-CH_2CH_3$ |
| 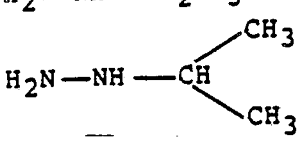 | 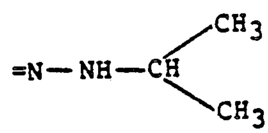 |

In the following Examples, the Active Ingredient is

(5R,6S,8R)sodium-2[2-(1,2,4-triazol-4-ylimino)propylthio]-6-(1-hydroxyethyl)-penem-3-carboxylate;
(5R,6S,8R)sodium-2-[2($N^2$-carbamoylhydrazono)propylthio]-6-(1-hydroxyethyl)penem-3-carboxylate;
or equivalent amount of a penem of compound of formula I.

Example 8

| Capsules No. | Ingredient | mg/tablet | mg/tablet |
|---|---|---|---|
| 1. | Active Ingredient | 250 | 500 |
| 2. | Lactose USP | 100 | 50 |
| 3. | Corn Starch, Food Grade | 50 | 43.5 |
| 4. | Microcrystalline Cellulose NF | 95 | 50 |
| 5. | Magnesium Stearate NF | 5 | 6.5 |
| | Total | 500 | 650 |

Method of Manufacture

Mix Items Nos. 1, 2, 3 and 4 in a suitable mixer for 10—15 minutes. Add Item No. 5 and mix for 1—3 minutes. Fill the mixture into suitable two-piece hard gelatin capsules using encapsulating machine.

Example 9

| Injectable Solution Ingredient | mg/ml | mg/ml |
|---|---|---|
| Active Ingredient | 100 | 500 |
| Methylparaben | 1.8 | 1.8 |
| Propylparaben | 0.2 | 0.2 |
| Sodium Bisulfite | 3.2 | 3.2 |
| Disodium Edetate | 0.1 | 1.5 |
| Sodium Sulfate | 2.6 | 2.6 |
| Water for Injection q.s. ad | 1.0 ml | 1.0 ml |

Method of Manufacture

1. Dissolve parabens in a portion (85% of the final volume) of the water for injection at 65—70°C.
2. Cool to 25—35°C. Charge and dissolve the sodium bisulfite, disodium edetate and sodium sulfate.
3. Charge and dissolve the active ingredient.
4. Bring the solution to final volume by adding water for injection.
5. Filter the solution through 0.22 micron membrane and fill into appropriate containers.
6. Terminally sterilize the units by autoclaving.

**Claims for the Contracting States: BE CH DE FR GB IT LI NL SE**

1. Compounds represented by the formula

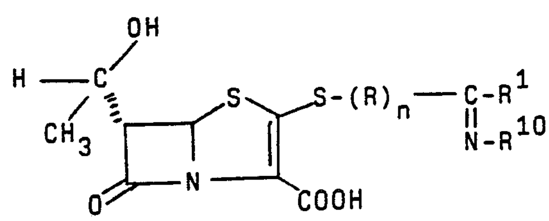

I

and pharmaceutically acceptable salts and pharmaceutically acceptable esters thereof, in racemic or optically active forms, wherein

n is one or two;

R represents

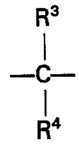

wherein $R^3$ and $R^4$ are independently selected from hydrogen, pyrrole, lower alkyl, hydroxy-lower alkyl, halo-lower alkyl, amino-lower alkyl, carbamido-lower alkyl, cyano-lower alkyl, carbamoyloxylower alkyl, carbamoyl-lower alkyl, carboxy-lower alkyl, heterocyclyl-lower alkyl wherein the heterocyclic moiety has 5 or 6 ring atoms, at least one of which is carbon, at least one of which is nitrogen, sulfur or oxygen, and the remaining ring atoms are independently selected from carbon, nitrogen, oxygen and sulfur;

$R^1$ represents hydrogen, loweralkoxycarbonylcarbonyl, carboxy, lower alkyl, hydroxy-lower alkyl, amino-lower alkyl, carboxy-lower alkyl, carbamoyloxy-lower alkyl, carbamoyl-lower alkyl, haloloweralkyl, cyano-lower alkyl, heterocyclyl, heterocyclyl-lower alkyl wherein the heterocyclic moieties have 5 or 6 ring atoms, at least one of which is carbon, at least one is nitrogen, oxygen or sulfur and the remaining ring

atoms are independently selected from oxygen, sulfur, nitrogen, and carbon;

$R^{10}$ represents 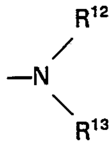 ;

$R^{12}$ and $R^{13}$ independently represent hydrogen, carbamoyl, lower alkyl, carboxy-lower alkyl, hydroxy-lower alkyl, amino-lower alkyl, imidazolyl-lower alkyl, triazolyl-lower alkyl, tetrazolyl-lower alkyl, pyridinium-lower alkyl, aryl, aryl-lower alkyl or $R^{12}$ and $R^{13}$ together with the N to which they are attached form a heterocyclic ring containing 5 or 6 atoms, at least one of which is carbon and the remaining ring atoms are independently selected from carbon, nitrogen, oxygen and sulfur wherein said heterocyclic moiety is unsubstituted or substituted with lower alkyl, hydroxy-lower alkyl, amino-lower alkyl, carboxy-lower alkyl, carboxyl, amino, hydroxy, imidazole-lower alkyl, triazole-lower alkyl, tetrazole-lower alkyl or pyridinium-lower alkyl, or when said heterocyclic moiety is 1,4-thiazane, said 1,4-thiazane moiety can also be substituted by two oxo-groups attached to the sulfur atom;

and wherein the term "lower" denotes $C_{1-6}$ and "aryl" denotes phenyl which is unsubstituted or substituted by substituents selected from lower alkyl, hydroxy-lower alkyl, carboxy-lower alkyl, carboxyl and amino.

2. Compounds of claim 1 wherein $R^1$ is hydrogen or lower alkyl.

3. Compounds of claims 1 or 2 wherein $R^3$ and $R^4$ are both hydrogen.

4. Compounds of any one of claims 1 to 3, wherein $R^{12}$ and $R^{13}$ independently represent hydrogen or carbamoyl, or $R^{12}$ and $R^{13}$ together with the nitrogen atom to which they are attached form a 5 or 6 membered heterocyclic ring wherein at least one ring atom is carbon and the remaining ring atoms are independently selected from carbon, oxygen, sulfur and nitrogen.

5. The compounds (5R,6S,8R)-2-[2-(1,2,4-triazol-4-ylimino)-propylthio]-6-(1-hydroxy-ethyl)penem-3-carboxylic acid,

(5R,6S,8R)-2-[2-[2-($N^2$-($N^2$-carbamoylhydrazono)-propylthio]-6-(1-hydroxyethyl)penem-3-carboxylic acid,

or their pharmaceutically acceptable salts and esters.

6. A pharmaceutical composition comprising an antibacterially effective amount of a compound as defined in any one of claims 1 to 5 in admixture with a pharmaceutically acceptable carrier therefor.

7. A process for preparing pharmaceutical compositions as defined in claim 6, characterized in that a compound of any one of claims 1 to 5 is admixed with a suitable pharmaceutical carrier.

8. A process for preparing compounds of formula I defined in claim 1, characterized in that a tautomeric compound of the formulae IXa and IXb

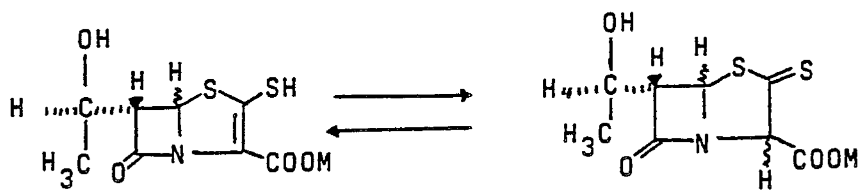

IX(a) IX(b)

is reacted with a compound of formula XII

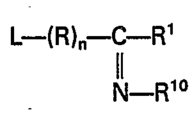

XII

wherein M is a carboxy protecting group, L is a leaving group and R, $R^1$, and $R^{10}$ are as defined in claim 1, to form a compound of formula XIII

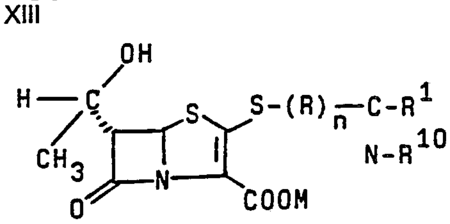

XIII

which is deprotected to yield a compound of formula I, followed, if desired, by converting the so obtained compound to a pharmaceutically acceptable salt or ester.

**Claims for the Contracting State: AT**

1. A process for preparing compounds represented by the formula

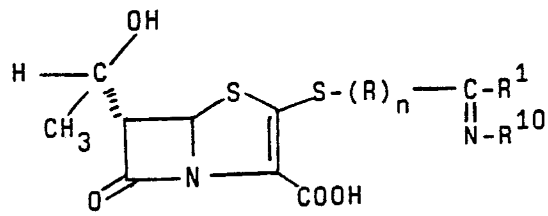

I

and pharmaceutically acceptable salts and pharmaceutically acceptable esters thereof, in racemic or optically active forms, wherein

n is one or two;

R represents

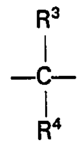

wherein $R^3$ and $R^4$ are independently selected from hydrogen, pyrrole, lower alkyl, hydroxy-lower alkyl, halo-lower alkyl, amino-lower alkyl, carbamido-lower alkyl, cyano-lower alkyl, carbamoyloxylower alkyl, carbamoyl-lower alkyl, carboxy-lower alkyl, heterocyclyl-lower alkyl wherein the heterocyclic moiety has 5 or 6 ring atoms, at least one of which is carbon, at least one of which is nitrogen, sulfur or oxygen, and the remaining ring atoms are independently selected from carbon, nitrogen, oxygen and sulfur;

$R^1$ represents hydrogen, loweralkoxycarbonylcarbonyl, carboxy, lower alkyl, hydroxy-lower alkyl, amino-lower alkyl, carboxy-lower alkyl, carbamoyloxy-lower alkyl, carbamoyl-lower alkyl, haloloweralkyl, cyano-lower alkyl, heterocyclyl, heterocyclyl-lower alkyl wherein the heterocyclic moieties have 5 or 6 ring atoms, at least one of which is carbon, at least one is nitrogen, oxygen or sulfur and the remaining ring atoms are independently selected from oxygen, carbon, nitrogen and sulfur;

$R^{10}$ represents 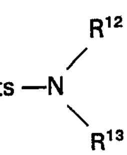 ;

$R^{12}$ and $R^{13}$ independently represent hydrogen, carbamoyl, lower alkyl, carboxy-lower alkyl, hydroxy-lower alkyl, amino-lower alkyl, imidazolyl-lower alkyl, triazolyl-lower alkyl, tetrazolyl-lower alkyl, pyridinium-lower alkyl, aryl, aryl-lower alkyl or $R^{12}$ and $R^{13}$ together with the N to which they are attached form a heterocyclic ring containing 5 or 6 atoms, at least one of which is carbon and the remaining ring atoms are independently selected from carbon, nitrogen, oxygen and sulfur wherein said heterocyclic moiety is unsubstituted or substituted with lower alkyl, hydroxy-lower alkyl, amino-lower alkyl, carboxy-lower alkyl, carboxyl, amino, hydroxy, imidazole-lower alkyl, triazole-lower alkyl, tetrazole-lower alkyl or pyridinium-lower alkyl, or when said heterocyclic moiety is 1,4-thiazane, said 1,4-thiazane moiety can also be substituted by two oxo-groups attached to the sulfur atom;

and wherein the term "lower" denotes $C_{1-6}$ and "aryl" denotes phenyl which is unsubstituted or substituted by substituents selected from lower alkyl, hydroxy-lower alkyl, carboxy-lower alkyl, carboxyl and amino

characterised in that a tautomeric compound of the formulae IXa and IXb

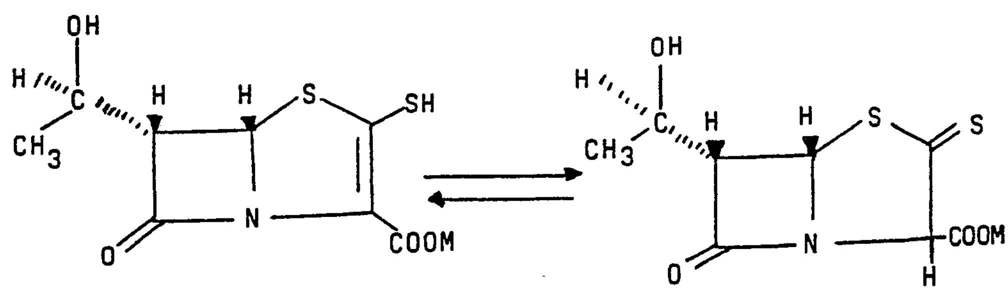

is reacted with a compound of formula XII

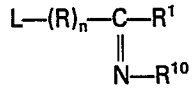

XII

wherein M is a carboxy protecting group, L is a leaving group and n, R, $R^1$, and $R^{10}$ are as defined above, to form a compound of formula XIII

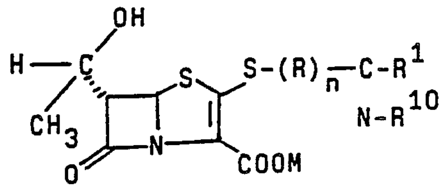

XIII

which is deprotected to yield a compound of formula I, followed, if desired, by converting the so-obtained compound to a pharmaceutically acceptable salt or ester.

2. The process of claim 1 wherein $R^1$ is hydrogen or lower alkyl.

3. The process of claims 1 or 2 wherein $R^3$ and $R^4$ are both hydrogen.

4. The process of any one of claims 1 to 3, wherein $R^{12}$ and $R^{13}$ independently represent hydrogen or carbamoyl, or $R^{12}$ and $R^{13}$ together with the nitrogen atom to which they are attached form a 5 or 6 membered heterocyclic ring wherein at least one ring atom is carbon and the remaining ring atoms are independently selected form carbon, oxygen, sulfur and nitrogen.

5. The process of claim 1 wherein the compound produced is (5R,6S,8R)-2-[2-(1,2,4-triazol-4-ylimino)propylthio]-6-(1-hydroxy-ethyl)penem-3-carboxylic acid,

(5R,6S,8R)-2-[2-($N^2$-carbamoylhydrazono)propylthio]-6-(1-hydroxyethyl)penem-3-carboxylic acid, or their pharmaceutically acceptable salts and esters.

6. A process for preparing a pharmaceutical composition characterized in that a compound as prepared by the process of any one of claims 1 to 5 is admixed with a pharmaceutically acceptable carrier.

**Patentansprüche für die Vertragsstaaten: BE CH DE FR GB IT LI NL SE**

1. Verbindungen der Formel

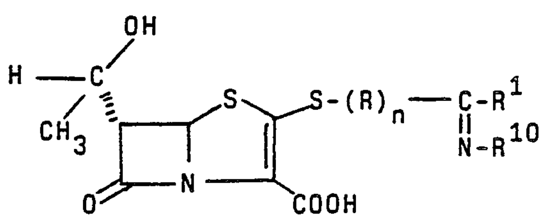

I

und pharmazeutisch annehmbare Salze und pharmazeutisch annehmbare Ester derselben in racemischer Form oder optisch aktiver Form, worin

n eins oder zwei ist,

R

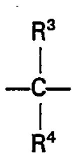 bezeichnet,

worin $R^3$ und $R^4$ unabhängig voneinander ausgewählt sind aus Wasserstoff, Pyrrol, Niederalkyl, Hydroxy-niederalkyl, Halogeno-niederalkyl, Amino-niederalkyl, Carbamido-niederalkyl, Cyano-niederalkyl, Carbamoyloxyniederalkyl, Carbamoyl-niederalkyl, Carboxy-niederalkyl, Heterocyclyl-niederalkyl, worin die heterocyclische Struktureinheit 5 oder 6 Ring-Atome hat, von denen wenigstens eines Kohlenstoff ist, wenigstens eines Stickstoff, Schwefel oder Sauerstoff ist und die übrigen Ring-Atome unabhängig aus Kohlenstoff, Stickstoff, Sauerstoff und Schwefel ausgewählt sind;

$R^1$ Wasserstoff, Niederalkoxycarbonylcarbonyl, Carboxy, Niederalkyl, Hydroxy-niederalkyl, Amino-niederalkyl, Carboxy-niederalkyl, Carbamoyloxy-niederalkyl, Carbamoyl-niederalkyl, Halogeno-niederalkyl, Cyano-niederalkyl, Heterocyclyl, Heterocyclyl-niederalkyl, worin die heterocyclische Struktureinheit 5 oder 6 Ring-Atome hat, von denen wenigstens eines Kohlenstoff ist, wenigstens eines Stickstoff, Sauerstoff oder

Schwefel ist und die übrigen Ring-Atome unabhängig aus Sauerstoff, Schwefel, Stickstoff und Kohlenstoff ausgewählt sind;

$R^{10}$

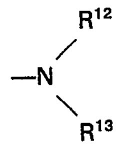

$R^{12}$ und $R^{13}$ unabhängig voneinander Wasserstoff, Carbamoyl, Niederalkyl, Alkoxy-niederalkyl, Carboxy-niederalkyl, Hydroxy-niederalkyl, Amino-niederalkyl, Imidazolylniederalkyl, Triazolyl-niederalkyl, Tetrazolyl-niederalkyl, Pyridinium-niederalkyl, Aryl, Aryl-niederalkyl, bezeichnen oder $R^{12}$ und $R^{13}$ zusammen mit dem N-Atom, an das sie gebunden sind, einen heterocyclischen Ring mit 5 oder 6 Atomen bilden, von denen wenigstens eines Kohlenstoff ist und die übrigen Ring-Atome unabhängig aus Kohlenstoff, Stickstoff, Sauerstoff und Schwefel ausgewählt sind, wobei die heterocyclische Struktureinheit unsubstituiert oder mit Niederalkyl, Hydroxyniederalkyl, Amino-niederalkyl, Carboxy-niederalkyl, Carboxyl, Amino-, Hydroxy, Imidazol-niederalkyl, Triazolyl-niederalkyl, Tetrazolyl-niederalkyl oder Pyridinium-niederalkyl substituiert ist oder, wenn die heterocyclische Struktureinheit 1,4-Thiazan ist, die 1,4-Thiazan-Struktureinheit auch durch zwei an das Schwefel-Atom gebundene Oxo-Gruppen substituiert sein kann;

und worin der Begriff "Nieder" $C_{1-6}$ bezeichnet und der Begriff "Aryl" Phenyl bezeichnet, das unsubstituiert oder durch Substituenten, ausgewählt aus Niederalkyl, Hydroxyniederalkyl, Carboxy-niederalkyl, Carboxyl und Amino substituiert ist.

2. Verbindungen nach Anspruch 1, worin $R^1$ Wasserstoff oder Niederalkyl ist.

3. Verbindungen nach Anspruch 1 oder 2, worin $R^3$ und $R^4$ beide Wasserstoff sind.

4. Verbindungen nach irgendeinem der Ansprüche 1 bis 3, worin $R^{12}$ und $R^{13}$ unabhängig voneinander Wasserstoff oder Carbamoyl bezeichnen oder $R^{12}$ und $R^{13}$ zusammen mit dem N-Atom, an das sie gebunden sind, einen 5- oder 6-gliedrigen heterocyclischen Ring bilden, von denen wenigstens ein Ring-Atom Kohlenstoff ist und die übrigen Ring-Atome unabhängig aus Kohlenstoff, Sauerstoff, Schwefel und Stickstoff ausgewählt sind.

5. Verbindungen (5R,6S,8R)-2-[2-(1,2,4-triazol-4-ylimino)propylthio]-6-(1-hydroxyethyl)penem-3-carbonsäure, (5R,6S,8R)-2-[2-($N^2$-carbamoylhydrazono)propylthio]-6-(1-hydroxyethyl)penem-3-carbonsäure, oder deren pharmazeutisch annehmbare Salze und Ester.

6. Pharmazeutische Zusammensetzung, umfassend eine antibakteriell wirksame Menge eine Verbindung nach irgendeinem der Ansprüche 1 bis 5 im Gemisch mit einem pharmazeutisch annehmbaren Träger dafür.

7. Verfahren zur Herstellung pharmazeutischer Zusammensetzungen nach Anspruch 6, dadurch gekennzeichnet, daß eine Verbindung nach irgendeinem der Ansprüche 1 bis 5 mit einem geeigneten pharmazeutischen Träger vermischt wird.

8. Verfahren zur Herstellung von Verbindungen der Formel (I) nach Anspruch 1, dadurch gekennzeichnet, daß eine tautomere Verbindung der Formeln IXa und IXb

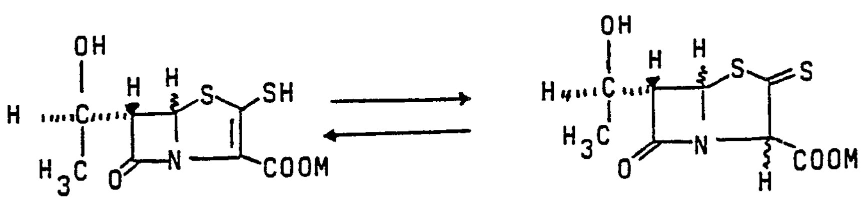

IX(a) IX(b)

mit einer Verbindung der Formel XII

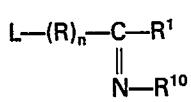

XII

umgesetzt wird, in der

M eine Schutzgruppe für eine Carboxy-Gruppe ist,

L eine abspaltbare Gruppe ist und

R, $R^1$ und $R^{10}$ die in Anspruch 1 angegebenen Bedeutungen haben, unter Bildung einer Verbindung der Formel XIII

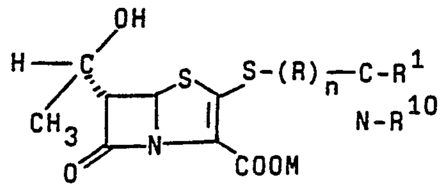

XIII

die von der Schutzgruppe befreit wird, wodurch eine Verbindung der Formel I erhalten wird, und daß anschließend gewünschtenfalls die auf diese Weise erhaltene Verbindung in ein pharmazeutisch annehmbares Salz oder einen pharmazeutisch annehmbaren Ester überführt wird.

**Patentansprüche für den Vertragsstaat: AT**

1. Verfahren zur Herstellungen von Verbindungen der Formel

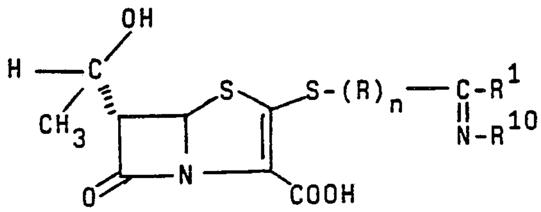

I

und pharmazeutisch annehmbarer Salze und pharmazeutisch annehmbare Ester derselben in racemischer Form oder optisch aktiver Form, worin

n eins oder zwei ist,

R

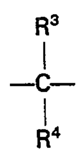 bezeichnet,

worin $R^3$ und $R^4$ unabhängig voneinander ausgewählt sind aus Wasserstoff, Pyrrol, Niederalkyl, Hydroxy-niederalkyl, Halogeno-niederalkyl, Amino-niederalkyl, Carbamido-niederalkyl, Cyano-niederalkyl, Carbamoyloxyniederalkyl, Carbamoyl-niederalkyl, Carboxy-niederalkyl, Heterocyclyl-niederalkyl, worin die heterocyclische Struktureinheit 5 oder 6 Ring-Atome hat, von denen wenigstens eines Kohlenstoff ist, wenigstens eines Stickstoff, Schwefel oder Sauerstoff ist und die übrigen Ring-Atome unabhängig aus Kohlenstoff, Stickstoff, Sauerstoff und Schwefel ausgewählt sind;

$R^1$ Wasserstoff, Niederalkoxycarbonylcarbonyl, Carboxy, Niederalkyl, Hydroxy-niederalkyl, Amino-niederalkyl, Carboxy-niederalkyl, Carbamoyloxy-niederalkyl, Carbamoyl-niederalkyl, Halogeno-niederalkyl, Cyano-niederalkyl, Heterocyclyl, Heterocyclyl-niederalkyl, worin die heterocyclische Struktureinheit 5 oder 6 Ring-Atome hat, von denen wenigstens eines Kohlenstoff ist, wenigstens eines Stickstoff, Sauerstoff oder Schwefel ist und die übrigen Ring-Atome unabhängig aus Sauerstoff, Schwefel, Stickstoff und Kohlenstoff ausgewählt sind;

$R^{10}$

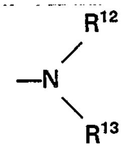

$R^{12}$ und $R^{13}$ unabhängig voneinander Wasserstoff, Carbamoyl, Niederalkyl, Alkoxy-niederalkyl, Carboxy-niederalkyl, Hydroxy-niederalkyl, Amino-niederalkyl, Imidazolylniederalkyl, Triazolyl-niederalkyl, Tetrazolyl-niederalkyl, Pyridinium-niederalkyl, Aryl, Aryl-niederalkyl, bezeichnen oder $R^{12}$ und $R^{13}$ zusammen mit dem N-Atom, an das sie gebunden sind, einen heterocyclischen Ring mit 5 oder 6 Atomen bilden, von denen wenigstens eines Kohlenstoff ist und die übrigen Ring-Atome unabhängig aus Kohlenstoff, Stickstoff, Sauerstoff und Schwefel ausgewählt sind, wobei die heterocyclische Struktureinheit unsubstituiert oder mit Niederalkyl, Hydroxyniederalkyl, Amino-niederalkyl, Carboxy-niederalkyl, Carboxyl, Amino-, Hydroxy, Imidazol-niederalkyl, Triazolyl-niederalkyl, Tetrazolyl-niederalkyl oder Pyridinium-niederalkyl substituiert ist oder, wenn die heterocyclische Struktureinheit 1,4-Thiazan ist, die 1,4-Thiazan-Struktureinheit auch durch zwei an das Schwefel-Atom gebundene Oxo-Gruppen substituiert sein kann;
und worin der Begriff "Nieder" $C_{1-6}$ bezeichnet und der Begriff "Aryl" Phenyl bezeichnet, das unsubstituiert oder durch Substituenten, ausgewählt aus Niederalkyl, Hydroxyniederalkyl, Carboxy-niederalkyl, Carboxyl und Amino substituiert ist, dadurch gekennzeichnet, daß eine tautomere Verbindung der Formeln IXa und IXb

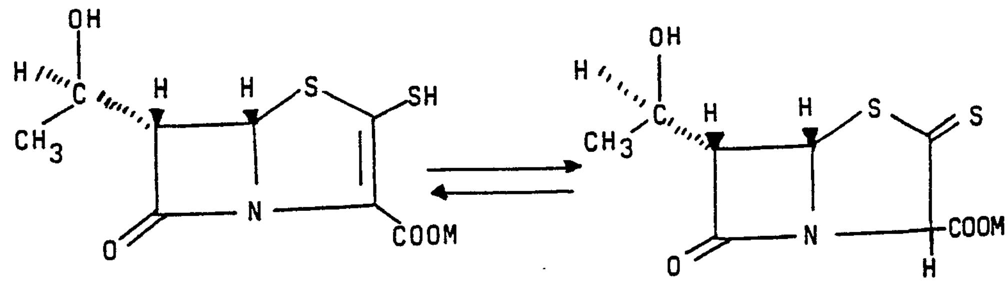

mit einer Verbindung der Formel XII

$$L-(R)_n-\underset{\underset{N-R^{10}}{\|}}{C}-R^1 \qquad XII$$

umgesetzt wird, in der
M eine Schutzgruppe für eine Carboxy-Gruppe ist,
L eine abspaltbare Gruppe ist und
R, $R^1$ und $R^{10}$ die in Anspruch 1 angegebenen Bedeutungen haben,
unter Bildung einer Verbindung der Formel XIII

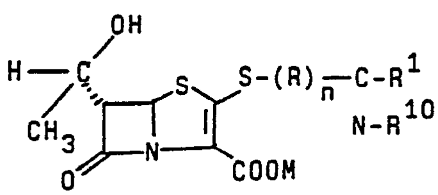

XIII

die von der Schutzgruppe befreit wird, wodurch eine Verbindung der Formel I erhalten wird, und daß anschließend gewünschtenfalls die auf diese Weise erhaltene Verbindung in ein pharmazeutisch annehmbares Salz oder einen pharmazeutisch annehmbaren Ester überführt wird.

2. Verfahren nach Anspruch 1, worin $R^1$ Wasserstoff oder Niederalkyl ist.

3. Verfahren nach Anspruch 1 oder 2, worin $R^3$ und $R^4$ beide Wasserstoff sind.

4. Verfahren nach irgendeinem der Ansprüche 1 bis 3, worin $R^{12}$ und $R^{13}$ unabhängig voneinander Wasserstoff oder Carbamoyl bezeichnen oder $R^{12}$ und $R^{13}$ zusammen mit dem N-Atom, an das sie gebunden sind, einen 5- oder 6-gliedrigen heterocyclischen Ring bilden, von denen wenigstens ein Ring-Atom Kohlenstoff ist und die übrigen Ring-Atome unabhängig aus Kohlenstoff, Sauerstoff, Schwefel und Stickstoff ausgewählt sind.

5. Verfahren nach Anspruch 1, worin die hergestellten Verbindungen
(5R,6S,8R)-2-[2-(1,2,4-triazol-4-ylimino)propylthio]-6-(1-hydroxyethyl)penem-3-carbonsäure,
(5R,6S,8R)-2-[2-($N^2$-carbamoylhydrazono)propylthio]-6-(1-hydroxyethyl)penem-3-carbonsäure, oder deren pharmazeutisch annehmbare Salze und Ester sind.

6. Verfahren zur Herstellung einer pharmazeutischen Zusammensetzung, dadurch gekennzeichnet, daß eine nach irgendeinem der Ansprüche 1 bis 5 hergestellte Verbindung mit einem geeigneten pharmazeutischen Träger vermischt wird.

**Revendications pour les Etats contractants: BE CH DE FR GB IT LI NL SE**

1. Composés représentés par la formule

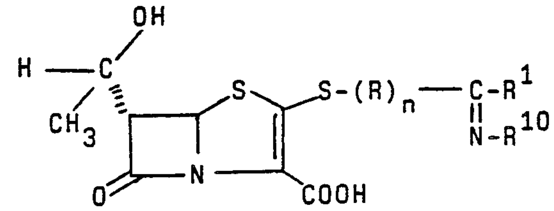

I

et leurs sels acceptables en pharmacie et esters acceptables en pharmacie, sous des formes racémiques ou optiquement actives, où

n est un ou deux;

R représente

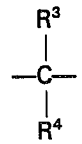

où $R^3$ et $R^4$ sont indépendamment choisi parmi hydrogène, pyrrole, alkyle inférieur, hydroxy-alkyle inférieur, halo-alkyle inférieur, amino-alkyle inférieur, carbamido-alkyle inférieur, cyano-alkyle inférieur, carbamoyloxy-alkyle inférieur, carbamoyl-alkyle inférieur, carboxy-alkyle inférieur, hétérocyclyl-alkyle inférieur, où le fragment hétérocyclique a 5 ou 6 atomes dans le noyau, dont au moins un est carbone, dont au moins un est azote, soufre ou oxygène, les atomes restants dans le noyau étant indépendamment choisis parmi carbone, azote, oxygène et soufre;

$R^1$ représente hydrogène, alcoxy inférieur-carbonyl-carbonyle, carboxy, alkyle inférieur, hydroxy-alkyle inférieur, amino-alkyle inférieur, carboxy-alkyle inférieur, carbamoyloxy-alkyle inférieur, carbamoyl-alkyle inférieur, halo-alkyle inférieur, cyano-alkyle inférieur, hétérocyclyle, hétérocyclyl-alkyle inférieur où les fragments hétérocycliques ont 5 ou 6 atomes dans le noyau, dont au moins un est carbone, dont au moins un est azote, oxygène ou soufre, les atomes restants du noyau étant indépendamment choisis parmi oxygène, soufre, azote et carbone;

$R^{10}$ représente 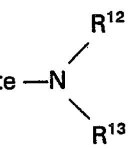 ;

$R^{12}$ et $R^{13}$ représentent indépendamment hydrogène, carbamoyle, alkyle inférieur, carboxy-alkyle inférieur, hydroxy-alkyle inférieur, amino-alkyle inférieur, imidazolyl-alkyle inférieur, triazolyl-alkyle inférieur, tétrazolyl-alkyle inférieur, pyridinium-alkyle inférieur, aryle, aryl-alkyle inférieur ou $R^{12}$ et $R^{13}$ avec le N auquel ils sont attachés forment un noyau hétérocyclique contenant 5 ou 6 atomes, dont au moins un est carbone, et les atomes restants du noyau sont indépendamment choisis parmi carbone, azote, oxygène et soufre où ledit fragment hétérocyclique est non substitué ou substitué par alkyle inférieur, hydroxy-alkyle inférieur, amino-alkyle inférieur, carboxy-alkyle inférieur, carboxyle, amino, hydroxy, imidazole-alkyle inférieur, triazole-alkyle inférieur, tétrazole-alkyle inférieur ou pyridinium-alkyle inférieur, ou quand ledit fragment hétérocyclique est 1,4-thiazane, ledit fragment 1,4-thiazane peut également être substitué par deux groupes oxo attachés à l'atome de soufre;

et où le terme "inférieur" désigne $C_{1-6}$ et "aryle" désigne phényle qui est non substitué ou substitué par des substituants choisis parmi alkyle inférieur, hydroxy-alkyle inférieur, carboxy-alkyle inférieur, carboxyle et amino.

2. Composés de la revendication 1 où $R^1$ est hydrogène ou alkyle inférieur.

3. Composés de la revendication 1 ou 2 où $R^3$ et $R^4$ sont tous deux de l'hydrogène.

4. Composés selon l'une des revendications 1 à 3 où $R^{12}$ et $R^{13}$ représentent indépendamment hydrogène ou carbamoyle, ou bien $R^{12}$ et $R^{13}$ avec l'atome d'azote auquel ils sont attachés forment un noyau hétérocyclique à 5 ou 6 membres où au moins un atome du noyau est du carbone, les atomes restants du noyau étant indépendamment choisis parmi carbone, oxygène, soufre et azote.

5. Les composés

acide (5R,6S,8R)-2-[2-(1,2,4-triazol-4-ylamino)propylthio]-6-(1-hydroxyéthyl)pénem-3-carboxylique,

acide (5R,6S,8R)-2-[2-($N^2$-carbamoylhydrazono)propylthio]-6-(1-hydroxyéthyl)pénem-3-carboxylique

ou leurs sels et esters acceptables en pharmacie.

6. Composition pharmaceutique comprenant une quantité antibactériennement efficace d'un composé tel que défini selon l'une des revendications 1 à 5 en mélange avec son véhicule acceptable en pharmacie.

7. Procédé de préparation de compositions pharmaceutiques selon la revendication 6, caractérisé en ce qu'un composé selon l'une des revendications 1 à 5 est mélange avec un véhicule pharmaceutique approprié.

8. Procédé de préparation de composés de la formule I définis à la revendication 1, caractérisé en ce qu'un composé tautomère des formules IXa et IXb

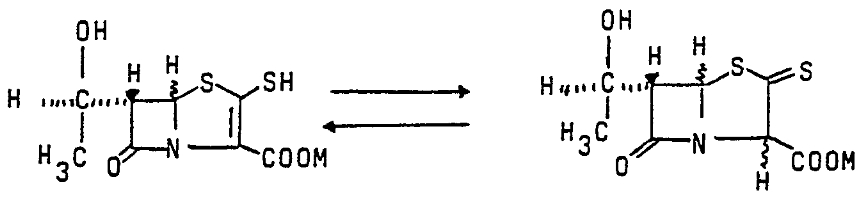

IX(a) IX(b)

réagit avec un composé de formule XII

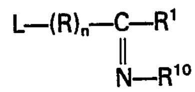

XII

où M est un groupe carboxy-protecteur, L est un groupe partant et R, $R^1$ et $R^{10}$ sont tels que définis à la revendication 1, pour former un composé de formule XIII

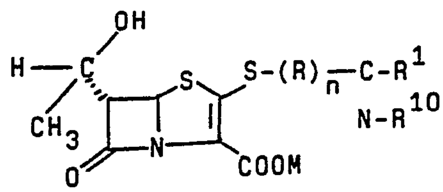

XIII

dont la protection est supprimée pour donner un composé de formule I, avec ensuite, si on le souhaite, conversion du composé ainsi obtenu en son sel ou ester acceptable en pharmacie.

**Revendications pour l'Etat contractant: AT**

1. Procédé de préparation de composés représentés par la formule

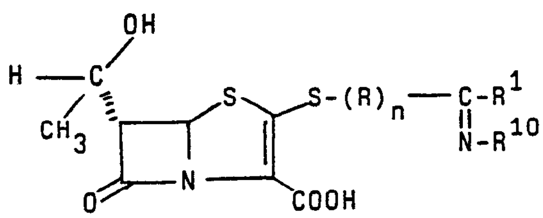

I

et leurs sels acceptables en pharmacie et esters acceptables en pharmacie, sous des formes racémiques ou optiquement actives, où

n est un ou deux;

R représente

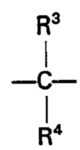

où $R^3$ et $R^4$ sont indépendamment choisi parmi hydrogène, pyrrole, alkyle inférieur, hydroxy-alkyle inférieur, halo-alkyle inférieur, amino-alkyle inférieur, carbamido-alkyle inférieur, cyano-alkyle inférieur, carbamoyloxy-alkyle inférieur, carbamoyl-alkyle inférieur, carboxy-alkyle inférieur, hétérocyclyl-alkyle

inférieur, où le fragment hétérocyclique a 5 ou 6 atomes dans le noyau, dont au moins un est carbone, dont au moins un est azote, soufre ou oxygène, les atomes restants dans le noyau étant indépendamment choisis parmi carbone, azote, oxygène et soufre;

$R^1$ représente hydrogène, alcoxy inférieur-carbonyl-carbonyle, carboxy, alkyle inférieur, hydroxy-alkyle inférieur, amino-alkyle inférieur, carboxy-alkyle inférieur, carbamoyloxy-alkyle inférieur, carbamoyl-alkyle inférieur, halo-alkyle inférieur, cyano-alkyle inférieur, hétérocyclyle, hétérocyclyl-alkyle inférieur où les fragments hétérocycliques ont 5 ou 6 atomes dans le noyau, dont au moins un est carbone, dont au moins un est azote, oxygène ou soufre, les atomes restants du noyau étant indépendamment choisis parmi oxygène, soufre, azote et soufre;

$R^{10}$ représente 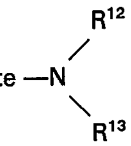 ;

$R^{12}$ et $R^{13}$ représentent indépendamment hydrogène, carbamoyle, alkyle inférieur, carboxy-alkyle inférieur, hydroxy-alkyle inférieur, amino-alkyle inférieur, imidazolyl-alkyle inférieur, triazolyl-alkyle inférieur, tétrazolyl-alkyle inférieur, pyridinium-alkyle inférieur, aryle, aryl-alkyle inférieur ou $R^{12}$ et $R^{13}$ avec le N auquel ils sont attachés forment un noyau hétérocyclique contenant 5 ou 6 atomes, dont au moins un est carbone, et les atomes restants du noyau sont indépendamment choisis parmi carbone, azote, oxygène et soufre où ledit fragment hétérocyclique est non substitué ou substitué par alkyle inférieur, hydroxy-alkyle inférieur, amino-alkyle inférieur, carboxy-alkyle inférieur, carboxyle, amino, hydroxy, imidazole-alkyle inférieur, triazole-alkyle inférieur, tétrazole-alkyle inférieur ou pyridinium-alkyle inférieur, ou quand ledit fragment hétérocyclique est 1,4-thiazane, ledit fragment 1,4-thiazane peut également être substitué par deux groupes oxo attachés à l'atome de soufre;

et où le terme "inférieur" désigne $C_{1-6}$ et "aryle" désigne phényle qui est non substitué ou substitué par des substituants choisis parmi alkyle inférieur, hydroxy-alkyle inférieur, carboxy-alkyle inférieur, carboxyle et amino caractérisé en ce qu'un composé tautomère des formules IXa et IXb

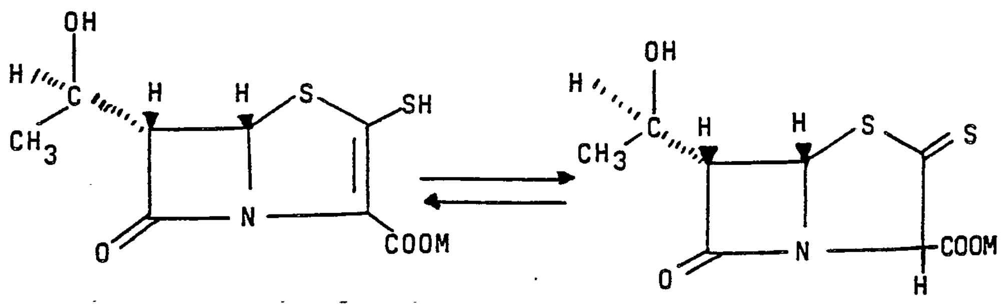

régit avec un composé de formule XII

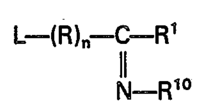

XII

où M est un groupe carboxy-protecteur, L est un groupe partant et R, $R^1$ et $R^{10}$ sont tels que définis ci-dessus, pour former un composé de formule XIII

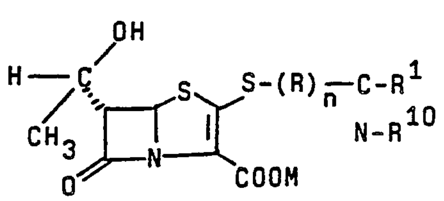

XIII

dont la protection est supprimée pour donner un composé de formule I, avec ensuite, si on le souhaite, conversion du composé ainsi obtenu en son sel ou ester acceptable en pharmacie.

2. Procédé de la revendication 1 où $R^1$ est hydrogène ou alkyle inférieur.

3. Procédé de la revendication 1 ou 2 où $R^3$ et $R^4$ sont tous deux hydrogène.

4. Procédé selon l'une des revendications 1 à 3 où $R^{12}$ et $R^{13}$ représentent indépendamment hydrogène ou carbamoyle, ou bien $R^{12}$ et $R^{13}$ avec l'atome d'azote auquel ils sont attachés forment un noyau hétérocyclique à 5 ou 6 membres où au moins un atome du noyau est le carbone, les atomes restants du noyau étant indépendamment choisis parmi carbone, oxygène, soufre et azote.

5. Procédé selon la revendication 1 où le composé produit est acide (5R,6S,8R)-2-[2-(1,2,4-triazol-4-ylimino)propylthio]-6-(1-hydroxyéthyl)pénem-3-carboxylique, acide (5R,6S,8R)-2-[2-($N^2$-carbamoylhydrazono)-propylthio]-6-(1-hydroxyéthyl)pénem-3-carboxylique, ou leurs sels et esters acceptables en pharmacie.

6. Procédé de préparation d'une composition pharmaceutique, caractérisé en ce qu'un composé tel que préparé par le procédé selon l'une des revendications 1 à 5 est mélangé avec un véhicule acceptable en pharmacie.